(19) Europäisches Patentamt
European Patent Office
Office européen des brevets

(11) EP 2 264 629 A1

(12) **EUROPEAN PATENT APPLICATION**

(43) Date of publication:
**22.12.2010 Bulletin 2010/51**

(51) Int Cl.:
**G06F 17/50** (2006.01)

(21) Application number: **10162670.3**

(22) Date of filing: **12.05.2010**

(84) Designated Contracting States:
**AL AT BE BG CH CY CZ DE DK EE ES FI FR GB GR HR HU IE IS IT LI LT LU LV MC MK MT NL NO PL PT RO SE SI SK SM TR**
Designated Extension States:
**BA ME RS**

(30) Priority: **12.06.2009 US 484021**

(71) Applicant: **Livermore Software Technology Corporation**
**Livermore CA 94551 (US)**

(72) Inventor: **Burke, Ritchie**
**Livermore, CA 94551 (US)**

(74) Representative: **Becker Kurig Straus**
**Patentanwälte**
**Bavariastrasse 7**
**80336 München (DE)**

(54) **Systems and methods of calculating electron dynamics using spin-dependent quantum trajectories**

(57) Methods and systems for calculating electron or ion dynamics using spin-dependent quantum trajectories are disclosed. According to one exemplary embodiment of the present invention, electron or ion dynamics are obtained by solving a set of equations for electrons' motion using spin-dependent quantum trajectories calculated from electron current with one equation for each electron in the atomic structure of a material of interest. The set of equations is time-dependent Schrödinger or Dirac equations for the nonrelativistic and relativistic regime, respectively. The electron current contains a set of spin-dependent terms that guarantee Fermi-Dirac statistics are obeyed. Steady state solution of the set of equations for electrons' motion is a set of wave functions in a three-dimensional space and in time. The spin-dependent quantum trajectories for each of the electrons are updated at each solution cycle, and therefore, mean-field approximation is avoided.

FIG. 1

100

START

102 — Receive an atomic structure definition of a material, in which electron or ion dynamics are desired to be calculated

104 — Establish a set of equations for electrons' motion using spin-dependent quantum trajectories from electron current, one equation per electron

106 — Obtain a solution, spectral (time-dependent) wave function, to the set of equations for the electrons' motion in time and space domain

108 — Check whether the solution has reached a steady state

110 — Continue for another solution using new trajectories

no

yes

112 — Calculate and output each electron's trajectories using the spectral wave function and corresponding eigenfunction

END

**Description**

**FIELD OF THE INVENTION**

The present invention generally relates to quantum mechanics, more particularly to systems and methods of calculating electron or ion dynamics using spin-dependent quantum trajectories.

**BACKGROUND OF THE INVENTION**

Quantum mechanics is a set of principles underlying the most fundamental known description of all physical systems at the submicroscopic scale (at the atomic level). Notable among these principles are both a dual wave-like and particle-like behavior of matter and radiation, and prediction of probabilities in situations where classical physics predicts certainties. Classical physics can be derived as a good approximation to quantum physics, typically in circumstances with large numbers of particles. Thus quantum phenomena are particularly relevant in systems whose dimensions are close to the atomic scale, such as molecules, atoms, electrons, protons and other subatomic particles. Quantum theory provides accurate descriptions for many previously unexplained phenomena such as black body radiation and stable electron orbits.

To simulate or mathematically describe quantum mechanics, theory with rigorous mathematical formulations have been developed by many physicists, for example, Paul Dirac, Erwin Schrödinger, etc. Schrödinger's equation describes how the quantum state of a physical system changes in time. In the standard interpretation of quantum mechanics, the quantum state, also called a wave function, is the most complete description that can be given to a physical system. In Dirac theory, the possible states of a quantum mechanical system are represented by unit vectors residing in a complex separable Hilbert space (variously called the "state space" or the "associated Hilbert space" of the system) well defined up to a complex number of norm 1 (the phase factor). In other words, the possible states are points in the projection of a Hilbert space, usually called the complex projective space. The exact nature of this Hilbert space is dependent on the system; for example, the state space for position and momentum states is the space of square-integrable functions, while the state space for the spin of a single proton is just the product of two complex planes. Each observable is represented by a maximally-Hermitian (precisely: by a self-adjoint) linear operator acting on the state space. Each eigenstate of an observable corresponds to an eigenvector of the operator, and the associated eigenvalue corresponds to the value of the observable in that eigenstate. If the operator's spectrum is discrete, the observable can only attain those discrete eigenvalues.

Traditionally, distance between two electrons within an atom has been calculated using approximate approaches. For example, one approximation based on molecular dynamics is valid for atoms not electrons, and for above certain temperature (e.g., a few thousand deg. Kelvin). Another approximation is based on density functional theory, which is limited to the quantum ground state not valid to excited states. Additionally, the prior art approaches for calculating interelectronic interaction under Coulomb's law use a mean-field model interaction (i.e., local density approximation), which is a statistical average of a quantum wave function. Furthermore, many of the prior art approaches do not account for the spin of electron, thereby requiring certain ad hoc correction. For example, Stationary-state quantum theory does not probe the Pauli' exclusion principle of one fermion can occupy a quantum state at a given time, which is enforced in an ad hoc manner by a manual anti-symmetrization of a many-body wave function. Finally, in certain prior art approaches, dynamics of electrons fail to maintain Fermi-Dirac statistics and hence all electrons become bosons instead of fermions.

Based on the aforementioned problems, drawbacks and shortcomings of the prior art approaches, it would, therefore be desirable to have a method and system for calculating electron or ion dynamics without the use of approximation such as mean-field averages or density-functionals.

**BRIEF SUMMARY OF THE INVENTION**

Methods and systems for calculating electron or ion dynamics using spin-dependent quantum trajectories are disclosed. According to one aspect of the present invention, electron or ion dynamics are obtained by solving a set of equations for electrons' motion using spin-dependent quantum trajectories calculated from electron current with one equation for each electron in the atomic structure of a material of interest. The set of equations is time-dependent Schrödinger or Dirac equations for the nonrelativistic and relativistic regime, respectively. The electron current contains a set of spin-dependent terms that guarantee Fermi-Dirac statistics are obeyed. Steady state solution of the set of equations for electrons' motion is a set of wave functions in a three-dimensional space and in time. The spin-dependent quantum trajectories for each of the electrons are updated at each solution cycle, and therefore, mean-field approximation is avoided.

According to another aspect, the solution of Schrödinger equations can be obtained using an implicit partial differential equation solver in time and space domain using an initial trial wave function. Dirac current can be expressed in terms of Schrödinger wave function and spin eignfunctions, when speed of electrons in the atomic structure is substantially slower than the speed of light.

According to yet another aspect, the steady state solution is determined by checking whether the calculation of the set of Schrödinger equations has converged to stationary eigenstates in an energy spectrum. Once the stationary eigenstates are verified, corresponding eigenfunctions can be obtained by performing inverse temporal Fourier transformation. New eigentrajectories can then be calculated thereafter. Similarly, the spectral trajectories of each electron can be calculated from the spectral wave function.

According to yet anther aspect, electron or ion's current and velocity can be calculated using wave functions that obey the time-independent Schrödinger equations, for which a time-dependent position can be calculated by time integration of the velocity. Since current, velocity and position are fields in space and time. Trajectories are then calculated from the quantum mean or expectation value of the position field. These trajectories behave like phase points in classical mechanics, although they are derived from quantum wave functions.

Other objects, features, and advantages of the present invention will become apparent upon examining the following detailed description of an embodiment thereof, taken in conjunction with the attached drawings.

## BRIEF DESCRIPTION OF THE DRAWINGS

These and other features, aspects, and advantages of the present invention will be better understood with regard to the following description, appended claims, and accompanying drawings as follows:

FIG. 1 is a flowchart illustrating an exemplary process of calculating electron or ion dynamics in the atomic structure of a material in accordance with one embodiment of the present invention;

FIG. 2 is a diagram showing quantum states and spin states of an exemplary electron whose dynamics can be calculated by a method, according to an embodiment of the present invention;

FIG. 3 is a diagram showing an exemplary energy spectrum of a helium atom according to an embodiment of the present invention;

FIG. 4 is a diagram showing an exemplary electron trajectories in a helium atom according to an embodiment of the present invention; and

FIG. 5 is a function diagram showing salient components of a computer, in which an embodiment of the present invention may be implemented.

## DETAILED DESCRIPTION

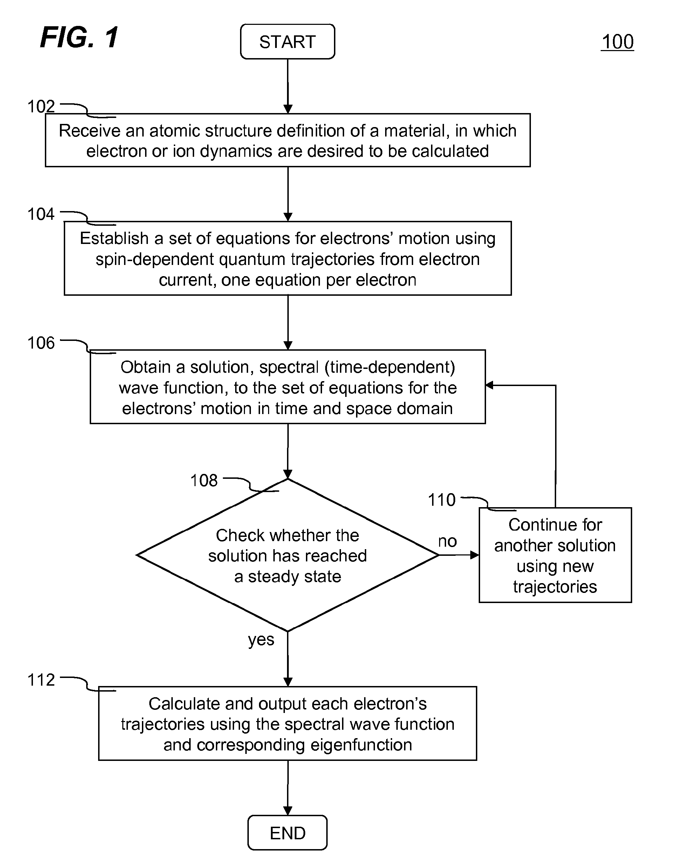

Embodiments of the present invention are discussed herein with reference to FIGS. 1-5. However, those skilled in the art will readily appreciate that the detailed description given herein with respect to these figures is for explanatory purposes as the invention extends beyond these limited embodiments.

Referring first to FIG. 1, which is a flowchart illustrating an exemplary process 100 of calculating electron or ion dynamics in the atomic structure of a material in accordance with one embodiment of the present invention. Process 100 may be implemented in software.

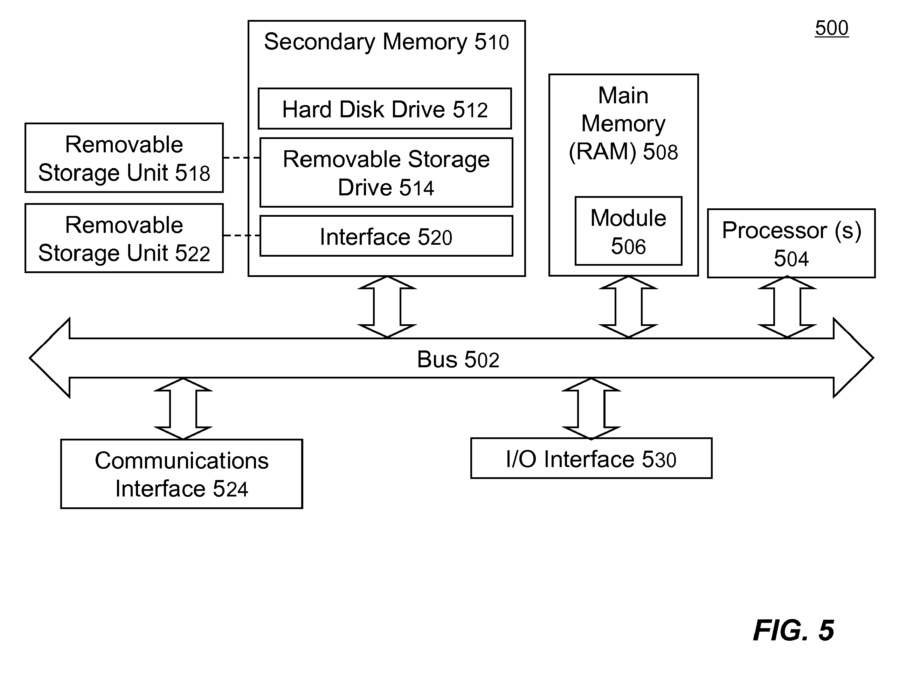

At step 102, process 100 starts by receiving, in a computer (e.g., computer system 500 in FIG. 5), an atomic structure definition of a material of interest, whose electron or ion dynamics are desired to be calculated. The material can be a metal alloy made of two or more different chemical elements or a pure metal consisting of single type of chemical elements. The atomic structure definition includes a plurality of electrons or ions, for example, aluminum atom contains 13 electrons. Electron dynamics comprises the interactions amongst all 13 electrons. Next, at step 104, a set of equations of electrons' motion is set up or established using spin-dependent quantum trajectories from electron current (e.g., based on the Dirac equation). Each of the equations for electrons' motion is for one electron (e.g., 13 equations for Aluminum). The set of equations of electrons' motion comprises time-dependent Schrödinger equations or Dirac equations for the nonrelativistic or relativistic regime, respectively.

In one embodiment, derivations of the set of equations for electrons' motion are described below. First, Dirac equation is written as:

$$i\hbar \frac{\partial \psi}{\partial t} = (-i\hbar c \vec{\alpha} \cdot \vec{\nabla} + \beta mc^2 + e\Phi)\psi \qquad (1)$$

such that the Dirac electron current emerges as:

$$\vec{j} = c\psi^{+}\vec{\alpha}\psi \tag{2}$$

$$\vec{\alpha} \quad = \quad \begin{bmatrix} 0 & \vec{\sigma} \\ \vec{\sigma} & 0 \end{bmatrix} \tag{3a}$$

$$\beta \quad = \quad \begin{bmatrix} I & 0 \\ 0 & I \end{bmatrix} \tag{3b}$$

$$\psi \quad = \quad \begin{bmatrix} \xi \\ \chi \end{bmatrix} \tag{3c}$$

where $\psi$ is wave function, $\hbar$ is reduced Planck's constant, c is the speed of light, m is the rest mass of the electron or ion, e is electron charge and $\Phi$ is the potential of the atom. Pauli's vector $\vec{\sigma}$ is written as unit vectors in the x, y, and z directions:

$$\vec{\sigma} \quad = \quad \hat{i}\begin{bmatrix} 0 & 1 \\ 1 & 0 \end{bmatrix} + \hat{j}\begin{bmatrix} 0 & -i \\ i & 0 \end{bmatrix} + \hat{k}\begin{bmatrix} 1 & 0 \\ 0 & -1 \end{bmatrix} \tag{3d}$$

Electron current obtained using Eq. (2) is the electron current in the relativistic regime.

In another embodiment, when the speed of electron is substantially slower than the speed of light, the inequality, $\dfrac{Ze^{2}}{\hbar c} << 1$ is atomic number, e.g., 13 for aluminum), is satisfied. The large and small Dirac components can be written in terms of Schrödinger' wave function $\psi_{S}$ and spin eigenfunctions $\chi_{m_{s}}$.

$$\xi \cong \psi_{S}\chi_{m_{s}} \tag{4a}$$

$$\chi \cong \frac{-i\hbar}{2mc}\vec{\sigma}\cdot\vec{\nabla}\psi_{S}\chi_{m_{s}} \tag{4b}$$

$$\chi_{m_s} = \chi_{\pm\frac{1}{2}} = \begin{bmatrix} 1 \\ 0 \end{bmatrix} , \begin{bmatrix} 0 \\ 1 \end{bmatrix} \qquad (4c)$$

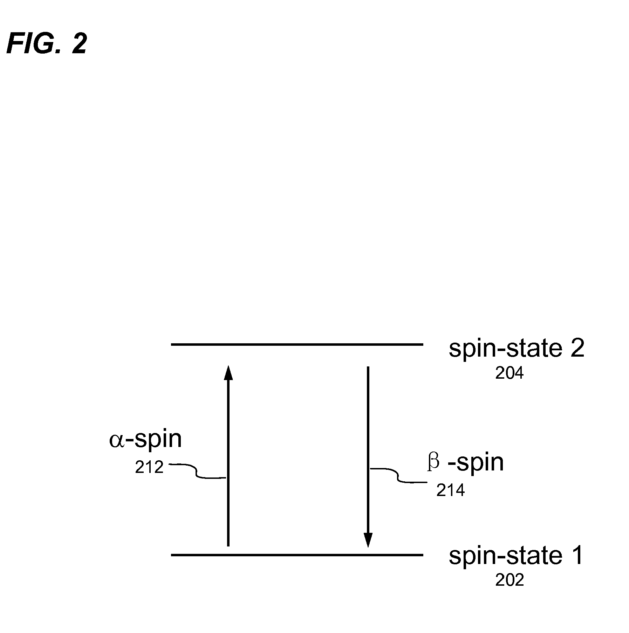

where $m_s$ is a spin-state of an electron. FIG. 2 is a diagram showing two quantum spin-states (i.e., "spin-state 1" 202 and "spin-state 2" 204 for higher and low energy levels, respectively). One example of the two spin-states is orbital "1s" and "2s". When an electron moves from "spin-state 1" 202 to "spin-state 2" 204, it is referred to as an "$\alpha$-spin" or "+1/2 spin" 212. The opposite movement of electron is a "$\beta$-spin" or "-1/2 spin" 214. Written out explicitly the electron current in Eq. (2) becomes electron current in the nonrelativistic regime.

$$\vec{j}_{NR} = c(\xi^+\vec{\sigma}\chi + \chi^+\vec{\sigma}\xi)$$

$$\vec{j}_{NR} \cong \frac{\hbar}{2m}[-i(\psi_S^*\vec{\nabla}\psi_S - \psi_S\vec{\nabla}\psi_S^*) + \psi_S^*\chi_{m_s}^+(\vec{\nabla}\times\vec{\sigma})\psi_S\chi_{m_s} + \psi_S\chi_{m_s}^+(\vec{\nabla}\times\vec{\sigma})\psi_S^*\chi_{m_s}] \qquad (5)$$

where the superscript daggers denote Hermitian conjugates such that $\vec{\sigma}^+ = \vec{\sigma}$ and the identity $(\vec{\sigma}\cdot\vec{A})(\vec{\sigma}\cdot\vec{B}) = \vec{A}\cdot\vec{B} + i\vec{\sigma}\cdot(\vec{A}\times\vec{B})$, from which, using standard vector identities, the identities useful in evaluating the current are obtained:

$$\vec{\sigma}(\vec{\sigma}\cdot\vec{\nabla}) = \vec{\nabla} + i(\vec{\nabla}\times\vec{\sigma}) \qquad (6a)$$

$$(\vec{\sigma}\cdot\vec{\nabla})\vec{\sigma} = \vec{\nabla} + i(\vec{\sigma}\times\vec{\nabla}) \qquad (6b)$$

Remarkably the spin-dependent terms (last two terms in Eq. (5) are of the same order in c, namely $c^0$, as the Schrödinger current, which is given by the spin-independent terms and which is the sole contribution using Schrödinger's equation. The current in the nonrelativistic regime can be written as follows:

$$\vec{j}_{NR}(\vec{r},t) =$$
$$\frac{\hbar}{m}[\text{Im}\,\psi_s^*(\vec{r},t)\vec{\nabla}\psi_s(\vec{r},t) \pm \hat{i}\,\text{Re}\,\psi_s^*(\vec{r},t)\frac{\partial}{\partial y}\psi_s(\vec{r},t) \mp \hat{j}\,\text{Re}\,\psi_s^*(\vec{r},t)\frac{\partial}{\partial x}\psi_s(\vec{r},t)] \qquad (7)$$

The upper and lower signs are for diagonal alpha-spin and beta-spin contributions, respectively. It is understood henceforth that $\psi_s(\vec{r},t)$ is the Schrödinger wave function. The first term on the right side of Eq. (7) is the Schrödinger current, which is spin-independent and vanishes for bound stationary eigenstates, which can always be chosen to be real. The next two terms for unit vectors in the "x" and "y" directions are spin-dependent contributions transverse to the quantization axis which are unique to Dirac theory. Velocity $\vec{\upsilon}(\vec{r},t)$ and position $\vec{s}(\vec{r},t)$ fields can be inferred from Eq. (7) as follows:

$$\vec{\upsilon}(\vec{r},t) = \frac{\vec{j}_{NR}(\vec{r},t)}{\psi_s^*(\vec{r},t)\psi_s(\vec{r},t)} \qquad (8a)$$

$$\vec{s}(\vec{r},t) = \int_0^t d\tau \vec{\upsilon}(\vec{r},\tau) \qquad (8b)$$

The electron's trajectory $\vec{r}(t)$ is then calculated from the expectation value of the position field.

$$\vec{r}(t) = \int d\vec{r}\psi_s^*(\vec{r},t)\vec{s}(\vec{r},t)\psi_s(\vec{r},t) \qquad (9)$$

It is noted that the wave function density in the denominator of Eq. (8a) is not cancelled by the wave function density in Eq. (9) due to the temporal integration in Eq. (8b), which archives the time history of the trajectory operator $\vec{s}$ $(\vec{r}, t)$ from $\tau=0$ to $\tau=t$. A time-dependent Schrödinger equation is written for each electron as follows:

$$(-\frac{\hbar^2}{2m}\nabla^2 - \frac{Ze^2}{r} + \sum_j \frac{e^2}{|\vec{r}-\vec{r}_j(t)|} - i\hbar\frac{\partial}{\partial t})\psi_s(\vec{r},t) = 0 \qquad (10)$$

where the summation term runs over all electrons excluding the reference electron whose motion is described by Eq. (10), "r" is the scalar distance between the reference electron and nucleus, "$\vec{r}$" is the reference electron's trajectories and the term "$|\vec{r} - \vec{r}_j(t)|$" is the exact relative trajectory between electron "j" and the reference electron.

It is clear from the foregoing development that the same quantum trajectory theory may be used for ion motion. Eq. (2) can be calculated for the spin state of a given ion point nucleus (for protons it would be the same as that given above for electrons such that the nuclear spin state-rotational state anti-symmetry of ortho-para-hydrogen would be described on an *ab initio* basis), and the Schrödinger equation (Eq. (10)) can be that for each ion interacting with the other ions by means of the appropriate electronic potentials depending on the quantum trajectories of the other ions. The present invention treats both electrons and ions quantum mechanically.

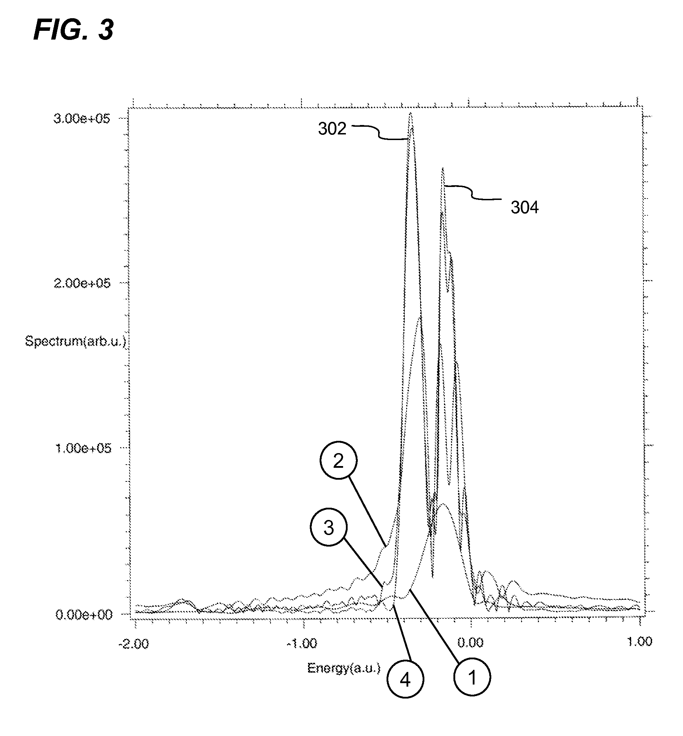

Referring now back to step 106, a solution (i.e., wave function $\psi_s(\vec{r},t)$) to the set of equations of electrons' motion (i.e., Eq. (10)) is obtained in a three-dimensional space (e.g., a Cartesian coordinate based space) and in time. In one embodiment, an implicit partial differential equation solver is used in conjunction with an initial trial wave function. The solution can be carries in the time domain as a number of solution cycles (i.e., time steps) After a predefined number of solution cycles, at decision 108, it is determined whether the solution has reached a steady state (e.g., stationary spectrum of states or stationary eigenstates) in an energy spectrum (e.g., exemplary helium atom energy spectrum in FIG. 3) of the wave function. Stationary eigenstates generally correspond to the energy level of electrons in the atomic structure. If "no", process 100 moves to step 110 to continue with another solution using the new trajectories (i.e., "$\vec{r}$" calculated from the wave function in Eq. (7)). Process 100 continues until the decision 108 becomes "yes", and moves to step 112. All electrons' trajectories can then calculated using both spectral wave function and corresponding eigenfunction before process 100 ends. The calculated trajectories are saved in a storage space coupled to the computer system and displayed to a monitor if desired.

To demonstrate the above method, an exemplary computation is carried out for a helium atom. The Schrödinger equations (Eq. (10)) are solved. It is noted that the Schrödinger contribution to the Dirac current in Eq. (7) does not vanish when Dirac current is evaluated using the time-dependent spectral wave functions from Eq. (10) since these are superpositions of eigenstates. Although the interelectronic interaction in Eq. (10) is time dependent, the solution has a stationary spectrum of states shown in FIG. 3 from which eigenfunctions can be calculated by inverse temporal Fourier transformation.

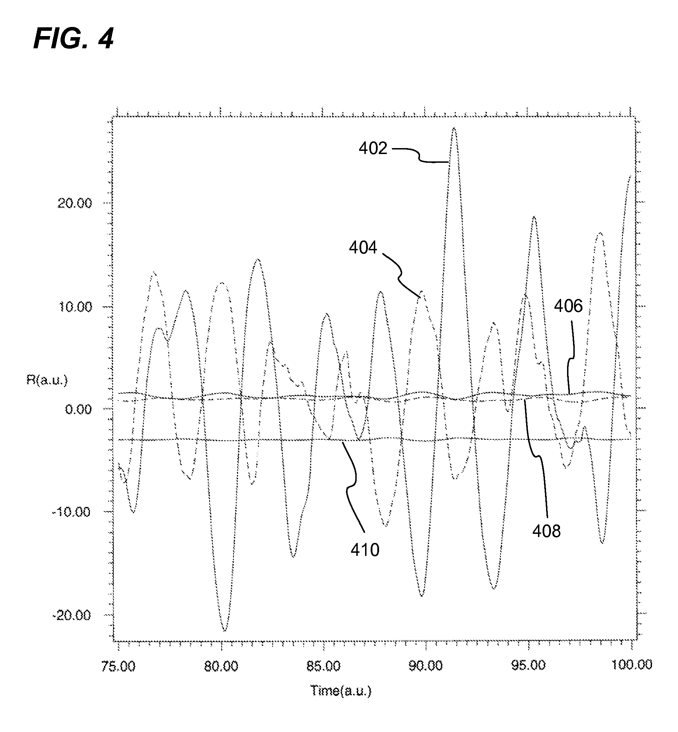

These eigenfunctions are then used in Eqs. (8a), (8b) and (9) to calculate new trajectories (i.e., eigen-trajectories). The many-electron energy is calculated from the expectation values of the sum of the two kinetic-energy and two nuclear potential-energy parts of the Hamiltonian and from the expectation values of the arithmetic average of the two interelectronic parts of the Hamiltonian. Notice that, although the interelectronic interaction is time dependent, the calculation nevertheless converges to stationary eigenstates shown in FIG. 3. This behavior is due to the periodicity of the electron motion shown in FIG. 4.

The physics of exchange-correlation is captured in the following way. If two electrons have opposite spin states, then these electrons correlate if they are assigned the same spatial orbital at initial time (FIG. 4). On the other hand if two electrons have the same spin state, then they do not correlate if they are assigned the same spatial orbital at initial time. These orbitals are rejected as unphysical. Two electrons with the same spin state correlate if they are assigned widely different spatial orbitals at initial time such that spatial orbitals which violate Pauli's exclusion principle are spectrally suppressed (FIG. 3).

Results of the helium atom computation are presented in FIGS. 3-4 and Table 1. FIG. 3 shows the convergence of the spectral "line" 302-304 for the excited orbital in the triplet excited state of helium and the almost complete extinction of the lower state. Numbers in circle indicate four solution convergence decisions (i.e., decision 108 of process 100 shown in FIG. 1) were made in the computation. The spectral peak appears grown higher at each subsequent convergence check. The line width of each spectral "line" 302-304 is due to the finite length of the temporal integration.

FIG. 4 shows later portion of time history of electron spectral trajectories 402-404 for the ground state calculated using the spectral wave function, eigentrajectories 406-408 calculated using the eigenfunctions and the two-electron energy 410. The eigenfunctions is associated with the spectral wave function by a "filter". The maximum excusions about the nucleus of the spectral trajectories are much larger than those of the eigen-trajectories, which reflect the excited-state content of the spectral wave functions. The envelope of the spectral trajectories gradually increases with time reflecting their continuum-state content. The flatness of the envelope of the eigentrajectories 406-408 shows that non-energy conserving errors in the integration of the trajectories have been reduced to an acceptable level.

It is noted that the spectral trajectories 402-404 are strongly correlated over most of the time history. This is due solely to the equal and opposite Dirac contributions to the current (Eq. (7)) for alpha- and beta-spin states, since each electron is assigned the same spatial orbital at initial time. The singlet excited state is similarly calculated using equal and opposite Dirac contributions and the same spatial orbital at initial time for both electrons except that in this case one of the electrons is assigned to the first-excited member of the spectrum of states generated from the time-dependent Schrödinger equation belonging to the electron which is assigned to the excited orbital. The triplet state is calculated using identical Dirac contributions to the current appropriate for the same spin state but with widely different orbitals assigned to the two electrons at initial time, as well as assignment of one of the electrons to the first excited member of the Schrödinger spectrum belonging to the electron.

Table 1 summarizes the computation results of energy levels for helium in atomic units (a.u.). The ground state calculation uses a 32x32x32 spatial mesh for a square computational box with edge length of 9.4981 a.u., while the excited state calculations use the same mesh for a box with edge length of 15.0773 a.u.

Table 1 - Energy Levels for Helium Atom

| State | Energy | Remarks |
|---|---|---|
| $1^1S$ | -3.00250 | 8001 temporal grid points for 0<t<100 a. u. |
| $2^1S$ | -2.14353 | 8001 temporal grid points for 0<t<200 a. u. |
| $2^3S$ | -2.17339 | 4001 temporal grid points for 0<t<200 a. u. |

According to one aspect, the present invention is directed towards one or more computer systems capable of carrying out the functionality described herein. An example of a computer system 500 is shown in FIG. 5. The computer system 500 includes one or more processors, such as processor 504. The processor 504 is connected to a computer system internal communication bus 502. Various software embodiments are described in terms of this exemplary computer system. After reading this description, it will become apparent to a person skilled in the relevant art(s) how to implement the invention using other computer systems and/or computer architectures.

Computer system 500 also includes a main memory 508, preferably random access memory (RAM), and may also include a secondary memory 510. The secondary memory 510 may include, for example, one or more hard disk drives 512 and/or one or more removable storage drives 514, representing a floppy disk drive, a magnetic tape drive, an optical disk drive, etc. The removable storage drive 514 reads from and/or writes to a removable storage unit 518 in a well-known manner. Removable storage unit 518, represents a floppy disk, magnetic tape, optical disk, flash memory, etc. which is read by and written to by removable storage drive 514. As will be appreciated, the removable storage unit 518 includes a computer recordable storage medium having stored therein computer software and/or data.

In alternative embodiments, secondary memory 510 may include other similar means for allowing computer programs or other instructions to be loaded into computer system 500. Such means may include, for example, a removable storage unit 522 and an interface 520. Examples of such may include a program cartridge and cartridge interface (such as that found in video game devices), a removable memory chip (such as an Erasable Programmable Read-Only Memory (EPROM), Universal Serial Bus (USB) flash memory, or PROM) and associated socket, and other removable storage units 522 and interfaces 520 which allow software and data to be transferred from the removable storage unit 522 to computer system 500. In general, Computer system 500 is controlled and coordinated by operating system (OS) software, which performs tasks such as process scheduling, memory management, networking and I/O services.

There may also be a communications interface 524 connecting to the bus 502. Communications interface 524 allows software and data to be transferred between computer system 500 and external devices. Examples of communications interface 524 may include a modem, a network interface (such as an Ethernet card), a communications port, a Personal Computer Memory Card International Association (PCMCIA) slot and card, etc.

The computer 500 communicates with other computing devices over a data network based on a special set of rules (i.e., a protocol). One of the common protocols is TCP/IP (Transmission Control Protocol/Internet Protocol) commonly used in the Internet. In general, the communication interface 524 manages the assembling of a data file into smaller packets that are transmitted over the data network or reassembles received packets into the original data file. In addition, the communication interface 524 handles the address part of each packet so that it gets to the right destination or intercepts packets destined for the computer 500.

In this document, the terms "computer program medium" and "computer recordable storage medium" are used to generally refer to media such as removable storage, a flash memory, and a hard disk installed in hard disk drive 512. These computer program products are means for providing software to computer system 500. The invention is directed to such computer program products.

The computer system 500 may also include an input/output (I/O) interface 530, which provides the computer system 500 to access monitor, keyboard, mouse, printer, scanner, plotter, and alike.

Computer programs (also called computer control logic) are stored as application modules 506 in main memory 508 and/or secondary memory 510. Computer programs may also be received via communications interface 524. Such computer programs, when executed, enable the computer system 500 to perform the features of the present invention as discussed herein. In particular, the computer programs, when executed, enable the processor 504 to perform features of the present invention. Accordingly, such computer programs represent controllers of the computer system 500.

In an embodiment where the invention is implemented using software, the software may be stored in a computer program product and loaded into computer system 500 using removable storage drive 514, hard drive 512, or communications interface 524. The application module 506, when executed by the processor 504, causes the processor 504 to perform the functions of the invention as described herein.

The main memory 508 may be loaded with one or more application modules 506 that can be executed by one or more processors 504 with or without a user input through the I/O interface 530 to achieve desired tasks. In operation, when at least one processor 504 executes one of the application modules 506, the results are computed and stored in the secondary memory 510 (i.e., hard disk or flash memory). The status of computation, analysis or solution (e.g., intermediate solutions of Schrödinger equations) is reported to the user via the I/O interface 530 either in a text or in a graphical representation (e.g., electron trajectories and energy spectrum).

Although the present invention has been described with reference to specific embodiments thereof, these embodiments are merely illustrative, and not restrictive of, the present invention. Various modifications or changes to the specifically disclosed exemplary embodiments will be suggested to persons skilled in the art. For example, whereas an exemplary verification uses Helium atom structure, other material or alloy can be used instead, for example, Aluminum or steel. Furthermore, whereas four convergence decisions or tests have been shown and described in the exemplary helium atom calculation, other numbers of test either higher or lower can be used to achieve the equivalent (e.g., 3, 5, 6, 7 ...). In summary, the scope of the invention should not be restricted to the specific exemplary embodiments disclosed herein, and all modifications that are readily suggested to those of ordinary skill in the art should be included within the spirit and purview of this application and scope of the appended claims.

**Claims**

1. A method of calculating electron or ion dynamics in a material's atomic structure comprising:

   receiving in a computer system an atomic structure definition of a material, the atomic structure definition includes a plurality of electrons or ions;
   establishing a set of equations for the electrons' motion with one equation for each of the plurality of the electrons, wherein the set of equations for the electrons' motion comprises a set of time-dependent Schrödinger equations in nonrelativistic regime or a set of time-dependent Dirac equations in relativistic regime, and each of the set of equations for the electrons' motion includes an inter-electron interaction between said each and every other electron of the plurality of the electrons, being represented by respective spin-dependent quantum trajectories of the plurality of the electrons, and each of the spin-dependent quantum trajectories is calculated from an electron current containing a set of spin-dependent terms that guarantee Fermi-Dirac statistics;
   solving the set of equations for the electrons' motion to generate a set of wave functions at a plurality of solution cycles in a three-dimensional space and in time, said each of the spin-dependent quantum trajectories for said each of the plurality of the electrons is updated at each of the solution cycles; and

storing a time history of the set of spin-dependent quantum trajectories representing the electron or ion dynamics of the material in a computer recordable storage medium coupled to the computer system and displaying the time history in an output device coupled to the computer system if desired.

2. The method of claim 1, wherein said each of the spin-dependent quantum trajectories is calculated from a set of position and velocity vectors of said each of the plurality of the electrons' position and velocity.

3. The method of claim 2, wherein the set of position and velocity vectors are derived from the electron current in accordance with a principle of classic dynamics.

4. The method of claim 1, wherein the inter-electron interaction includes a combination of attractions and repulsions among the plurality of the electrons in accordance with Pauli's exclusion principle.

5. The method of claim 1, wherein the set of spin-dependent terms of the electron current is in form of a set of Pauli's vectors.

6. The method of claim 1, wherein the material comprises a metal alloy made of at least two different chemical elements.

7. The method of claim 1, wherein the material comprises a metal made of a same type of chemical elements.

8. The method of claim 1, wherein said solving the set of equations for the electrons' motion further comprises providing a set of trial wave functions at onset.

9. The method of claim 1, wherein said solving the set of equations for the electrons' motion further comprises periodically checking an energy spectrum of the wave functions to determine whether a steady-state solution has been reached.

10. A system for calculating electron or ion dynamics in atomic structure of a material comprising:

a main memory for storing computer readable code for an application module;
at least one processor coupled to the main memory, said at least one processor executing the computer readable code in the main memory to cause the application module to perform operations by a method of:
receiving an atomic structure definition of a material, the atomic structure definition includes a plurality of electrons or ions;
establishing a set of equations for the electrons' motion with one equation for each of the plurality of the electrons, wherein the set of equations for the electrons' motion comprises a set of time-dependent Schrödinger equations in nonrelativistic regime or a set of time-dependent Dirac equations in relativistic regime, and each of the set of equations for the electrons' motion includes an inter-electron interaction between said each and every other electron of the plurality of the electrons, being represented by respective spin-dependent quantum trajectories of the plurality of the electrons, and each of the spin-dependent quantum trajectories is calculated from an electron current containing a set of spin-dependent terms that guarantee Fermi-Dirac statistics;
solving the set of equations for the electrons' motion to generate a set of wave functions at a plurality of solution cycles in a three-dimensional space and in time, said each of the spin-dependent quantum trajectories for said each of the plurality of the electrons is updated at each of the solution cycles; and
storing a time history of the set of spin-dependent quantum trajectories representing the electron or ion dynamics of the material in a computer recordable storage medium coupled to the system and displaying the time history in an output device coupled to the system if desired.

11. The system of claim 10, wherein the inter-electron interaction includes a combination of attractions and repulsions among the plurality of the electrons in accordance with Pauli's exclusion principle.

12. The system of claim 10, wherein said solving the set of equations for the electrons' motion further comprises providing a set of trial wave functions at onset.

13. A computer recordable storage medium containing instructions for controlling a computer system for calculating electron or ion dynamics in atomic structure of a material by a method comprising:

receiving in a computer system an atomic structure definition of a material, the atomic structure definition includes a plurality of electrons or ions;

establishing a set of equations for the electrons' motion with one equation for each of the plurality of the electrons, wherein the set of equations for the electrons' motion comprises a set of time-dependent Schrödinger equations in nonrelativistic regime or a set of time-dependent Dirac equations in relativistic regime, and each of the set of equations for the electrons' motion includes an inter-electron interaction between said each and every other electron of the plurality of the electrons, being represented by respective spin-dependent quantum trajectories of the plurality of the electrons, and each of the spin-dependent quantum trajectories is calculated from an electron current containing a set of spin-dependent terms that guarantee Fermi-Dirac statistics;

solving the set of equations for the electrons' motion to generate a set of wave functions at a plurality of solution cycles in a three-dimensional space and in time, said each of the spin-dependent quantum trajectories for said each of the plurality of the electrons is updated at each of the solution cycles; and

storing a time history of the set of spin-dependent quantum trajectories representing the electron or ion dynamics of the material in a computer recordable storage medium coupled to the computer system and displaying the time history in an output device coupled to the computer system if desired.

14. The computer recordable storage medium of claim 13, wherein said each of the spin-dependent quantum trajectories is calculated from a set of position and velocity vectors of said each of the plurality of the electrons' position and velocity.

15. The computer recordable storage medium of claim 14, wherein the set of position and velocity vectors are derived from the electron current in accordance with a principle of classic dynamics.

## *FIG. 1*

START                    100

102 — Receive an atomic structure definition of a material, in which electron or ion dynamics are desired to be calculated

104 — Establish a set of equations for electrons' motion using spin-dependent quantum trajectories from electron current, one equation per electron

106 — Obtain a solution, spectral (time-dependent) wave function, to the set of equations for the electrons' motion in time and space domain

108 — Check whether the solution has reached a steady state

no → 110 — Continue for another solution using new trajectories

yes

112 — Calculate and output each electron's trajectories using the spectral wave function and corresponding eigenfunction

END

*FIG. 2*

spin-state 2
204

$\alpha$-spin
212

$\beta$ -spin
214

spin-state 1
202

# FIG. 3

## FIG. 4

**FIG. 5**

Europäisches
Patentamt

European
Patent Office

Office européen
des brevets

## PARTIAL EUROPEAN SEARCH REPORT

under Rule 62a and/or 63 of the European Patent Convention.
This report shall be considered, for the purposes of
subsequent proceedings, as the European search report

Application Number

EP 10 16 2670

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| L | The claimed subject-matter, with due regard to the description and drawings in accordance with Art. 92 EPC, relates to processes comprised in the list of subject-matter and activities excluded from patentability under Art. 52(2) and (3) EPC. The information technology employed as an enabler for carrying out said processes is conventional. Its use for carrying out non-technical processes forms part of common general knowledge and it was widely available to everyone at the date of filing of the present application. No documentary evidence is therefore considered necessary.<br>----- | 1-15 | INV.<br>G06F17/50 |

TECHNICAL FIELDS
SEARCHED        (IPC)

G06F

### INCOMPLETE SEARCH

The Search Division considers that the present application, or one or more of its claims, does/do
not comply with the EPC so that only a partial search (R.62a, 63) has been carried out.

Claims searched completely :

Claims searched incompletely :

Claims not searched :

Reason for the limitation of the search:

see sheet C

| Place of search | Date of completion of the search | Examiner |
|---|---|---|
| Munich | 8 November 2010 | Wellisch, J |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04E07)

Europäisches
Patentamt

European
Patent Office

Office européen
des brevets

**INCOMPLETE SEARCH
SHEET C**

**Application Number**

EP 10 16 2670

Claim(s) searched incompletely:
        1-15

Reason for the limitation of the search (non-patentable invention(s)):

Claims 1-15 comprise subject matter that falls within the provisions of
Article 52 (2)(a) and (3) EPC; this for the following reasons:
The subject matter of claims 1-15 relates partly to a scientific theory
as such. Claim 1-15 specify a set of mathematical features relating to
the calculation of electron or ion dynamics in a material's atomic
structure, these features providing the theoretical framework for said
calculation.
While such a scientific formulation could be used to solve a number of
different technical problems, the claims fail to give the theory a
concrete technological context, and thereby fail to propose a solution to
a concrete technical problem. Instead, the theory is claimed in full
generality.
Under the provisions of Article 52 (2)(a) and (3) EPC such subject matter
is excluded from patentability and can hence not be used in support of
inventive step.
As the subject matter of claims 1-15 that does not fall under the
provisions of Article 52 (2) and (3) EPC is such as to be compatible with
the use of a general purpose computer, a meaningful search cannot be
performed with regards to these features.
In the response to a communication under Rule 63 EPC, the applicant
indicated that the search should be directed towards computer implemented
embodiments of the claims. The search was restricted to the technical
features in the claim taking this restriction into account.